# EUROPEAN PATENT SPECIFICATION

(11) **EP 2 594 266 B1**
(45) Date of publication and mention of the grant of the patent: **30.07.2014**
(21) Application number: 11009132.9
(22) Date of filing: 17.11.2011
(51) Int. Cl.: A61K 31/192, A61K 47/02, A61K 9/00

(54) **Pharmaceutical solid compositions containing ibuprofen salts**
Pharmazeutische Feststoffzusammensetzungen mit Ibuprofensalzen
Compositions solides pharmaceutiques contenant des sels ibuprofènes

(43) Date of publication of application: 22.05.2013
(73) Proprietor: Zambon S.p.A., 20091 Bresso MI (IT)
(72) Inventor: Moretto, Alberto, 35020 Ponte San Nicolò (Padova) (IT); De Lazzari, Alessandra, 35142 Padova (IT); Dicorato, Lucia, 35131 Padova (IT)
(74) Representative: Allaix, Roberto

(56) References cited:
- US-A1- 2007 265 344
- P. A.S. BRESLIN ET AL: "Ibuprofen as a Chemesthetic Stimulus: Evidence of a Novel Mechanism of Throat Irritation", CHEMICAL SENSES, vol. 26, no. 1, 1 January 2001 (2001-01-01), pages 55-65, XP55021161, ISSN: 0379-864X, DOI: 10.1093/chemse/26.1.55

## Description

### FIELD OF THE INVENTION

The present invention relates to pharmaceutical solid compositions containing ibuprofen salts, which do not cause sensory irritation to the oral cavity especially to the back of the mouth and throat, when swallowed in liquid pharmaceutical oral solutions, particularly when ibuprofen is administered at high dosage, while maintaining fast therapeutic onset.

### BACKGROUNG OF THE INVENTION

Ibuprofen, i.e. 2-(4-isobutylphenyl) propionic acid, is a known active pharmaceutical ingredient with analgesic, antiphlogistic and antipyretic properties, that in particular is employed for the treatment of inflammatory diseases and against pain, such as rheumatic diseases, headaches, migraines, toothaches, back aches, muscle pain, postoperative pain and the like.

The therapeutically effective form is the S(+)-ibuprofen, whereas the R(-)-enantiomer is practically ineffective, but converts in the body partly into the effective S(+)-form. Although in the last years some preparations containing ibuprofen in the S(+)-form have become available on the market, ibuprofen is still employed mostly in racemic form.

It is well known that ibuprofen salts have a very bitter taste and produce throat irritation with burning sensation, in particular when administered in high dosage forms, for example when the salt contains the equivalent to 400 mg, 600 mg or 800 mg of the ibuprofen dissolved in a suitable liquid for dilution, to be swallowed as a liquid solution.

Beside the peculiar unpleasant taste, subjects who assume oral composition comprising ibuprofen salts perceive uncomfortable sensory irritation towards the back of the mouth and the throat, typically described as sensations of burning, itching and tickling. In addition, for some subjects, the assumption of ibuprofen salts in a liquid oral dosage form triggers cough.

This problem is particularly relevant for pediatric pharmaceutical preparations, in which even the slightest residue of irritation may imply a total refusal of the drug for the child.

Said irritation associated with ibuprofen-based products is therefore an issue, which impacts on patient compliance, commercial success and product life cycle management.

Some attempts have been made to solve the above-identified irritation problem. Although strategies based on application of physical barriers between the active product ingredient and the oral mucosa (i.e. colloidal system with increased viscosity), reduction of the active product ingredient solubility in a suitable pH environment or by encapsulation techniques (granulation, coating, micro-encapsulation, etc.), complexation of active product ingredient with insoluble polymers or insoluble ion exchange resins, inclusion complexes with cyclodestrins, have been applied, most of the above approaches involve physical or chemical modification of the active product ingredient, which influence the regulatory status of the same, the dosage form, and may reduce bioavailability or onset of action. Furthermore, such methods are expensive and may increase manufacturing complexity.

Also, the classical approach of adding to the composition known irritants, which over-stimulate the trigeminal nerve in order to confuse the brain perception, show limited effectiveness with ibuprofen, and is always related to individual sensitivity and perception.

US 5262179 (US' 179) discloses non-effervescent water soluble compositions of water soluble ibuprofen salts, in which the unpleasant taste of the salt containing the equivalent to 200 mg and 400 mg of ibuprofen dissolved in 100 ml of water was improved by the addition of a molar excess of weak bases, such as bicarbonates, mono hydrogen phosphates or tri-basic citrates. Most of the examples clearly demonstrated that only a molar excess of the weak base was able to render the objectionable taste and burning sensation normally associated with ibuprofen salts, especially sodium ibuprofen, substantially undetectable in the resulting solution by most patients. According to US'179, sodium bicarbonate was the best choice to improve the taste and, usually, said taste-masking compound is present in a molar excess to the ibuprofen salt calculated as free ibuprofen. Furthermore, on column 3 lines 39-42 of US' 179 is described in detail that strong bases such as, for example, alkali metal carbonates and phosphates cannot be used to improve the taste because, in potential taste masking amounts, the resultant aqueous solution has an unacceptably high pH for oral administration, being the pH of the obtained solution too high for mucosal tolerability. In addition, increased sodium concentration due to a molar excess of sodium bicarbonate to ibuprofen may be harmful for some subjects, especially patients who need low sodium diet.

Despite various efforts to find effective means to eliminate the burning feeling in the mouth and throat after ingestion of ibuprofen salts, there is still a need of solid preparations of ibuprofen salts, particularly when ibuprofen salts contain the equivalent of ibuprofen at high dosage, suitable for administration as liquid oral formulations, that do not produce oral sensory irritation and avoid excessive metal ions content in solution (for example sodium or potassium), meanwhile providing a rapid onset of action.

The present invention fulfills such a need by providing pharmaceutically and commercially acceptable solid pharmaceutical compositions useful for the preparation of pharmaceutical formulations for oral use of ibuprofen salts, in particular ibuprofen salts containing the equivalent of ibuprofen at high dosages such as, for example, 400 mg, 600 mg and 800 mg, to be administered as liquid preparations that prevent irritation in the oral cavity, so as to make them more pleasant and to optimize the better patient's compliance. In particular, drinkable water solutions obtainable dissolving the solid pharmaceutical compositions of ibuprofen salts according to the present invention could represent, in many cases, particularly advantageous means of administration, especially for children, old people and those who for whatever reason cannot or prefer not to swallow solid dosage forms of drugs.

### DESCRIPTION OF THE INVENTION

The present inventors have surprisingly discovered that the addition of a strong pharmaceutically acceptable base, totally removes oral cavity irritation, when the solid composition comprising a ibuprofen salt is dissolved and swallowed as a liquid dosage formulation, especially when said drug is administered at high dosages, such as, for example, ibuprofen equal value from 400 mg to 800 mg.

In particular, the present inventors have found that, in order to eliminate the uncomfortable sensory irritation towards the back of the mouth and the throat, the molar amount of a strong base to ibuprofen salt must be in defect, namely the molar ratio of a strong base to the ibuprofen salt in a solid composition may range from 0.01:1 to 0.8:1, preferably from 0.5:1 to 0.8:1, so that when said solid composition is dissolved in a suitable liquid for dilution, such as, for example, an aqueous solution, particularly drinkable water, a final solution pH in the range from 9 to 9.5 is obtained. This pH range is compatible with a pharmaceutical liquid oral administration.

This specific pH ranges from 9.0 to 9,5 is easy achievable using very small amount of strong base while it is very difficult to obtain using weak base. For same weak base, for example sodium bicarbonate, pH range form 9 to 9.5 is impossible to achive, for other weak bases is possible to obtain but only using very large amount of weak base to ibuprofen.

It is therefore an object of the present invention a pharmaceutical, non-effervescent, solid composition comprising a mixture of a pharmaceutically effective amount of a ibuprofen salt and a pharmaceutically acceptable strong base in a molar ratio of from 1:0.01 to 1:0.8; preferably from 1:0.5 to 1:0.8, said composition being such that, when dissolved in a suitable liquid for dilution, such as, for example, an aqueous solution, particularly drinkable water, imparts a pH value ranging from 9 to 9.5, preferably a pH value of 9.2, to a said liquid solution. In a preferred aspect, the pharmaceutically effective amount of ibuprofen salt contains the equivalent of high dosage ibuprofen such as, 400 mg, 600 mg or 800 mg.

In a further aspect, the present invention refers to the use of a pharmaceutical, non-effervescent, solid composition for preparing a liquid solution, preferably an aqueous solution, more preferably drinkable water, having a pH value ranging from 9 to 9.5, preferably a pH value of 9.2, wherein said composition comprises a mixture of a pharmaceutically effective amount of a ibuprofen salt and a pharmaceutically acceptable strong base in a molar ratio of from 1:0.01 to 1:0.8, preferably from 1:0.5 to 1:0.8, and wherein the solid composition is dissolvable in a suitable liquid for dilution, preferably an aqueous solvent, more preferably drinkable water, having a volume of from 25 ml to 100 ml, preferably a volume of 50 ml.

A non-irritant liquid formulation obtainable by dissolving the above composition in a suitable liquid for dilution preferably an aqueous solution, more preferably drinkable water, is also encompassed by the scope of the present invention. Although the amount of the suitable liquid for dilution to be used is not binding, a liquid formulation according to the present invention can be typically prepared by dissolving from 2000 mg to 5000 mg of said composition, preferably from 3000 mg to 4000 mg, in a volume ranging from 50 ml to 100 ml, preferably 50 ml of drinkable water.

With the term "non-irritant liquid formulation" it is meant a liquid formulation that when swallowed does not cause unpleasant feeling of irritation of the oral cavity.

A further object of the present invention is represented by a method for eliminating unpleasant feeling of irritation of the oral cavity that usually follows the ingestion of ibuprofen salts as a liquid solution, by dissolving a composition according to the present invention in a suitable liquid for dilution, preferably an aqueous solution, more preferably drinkable water.

The pharmaceutical composition according to the invention can be useful for the preparation of solid non-effervescent pharmaceutical formulations for oral use comprising a mixture of a pharmaceutically effective amount of a ibuprofen salt and a pharmaceutically acceptable strong base in a molar ratio of from 1:0.01 to 1:0.8; the formulation being such that, if dissolved in a liquid solution, preferably an aqueous solution, more preferably drinkable water, the resultant solution has a pH value ranging from 9 to 9.5, preferably 9.2.

According to the present invention, an ibuprofen salt is selected from, for example, L-arginine, L-lysine, sodium or potassium ibuprofen salt, L-arginine ibuprofen salt being the preferred one. Said salts may be previously formed or, alternatively, directly prepared in situ according to techniques known to the skilled person in the art.

According to the present invention, a strong base is selected from, for example, an alkaline metal carbonate such as, for example, sodium or potassium carbonate; an alkaline metal hydroxide such as, for example, sodium or potassium hydroxide; a tribasic metal phosphate such as, for example, sodium and potassium tribasic phosphate, sodium carbonate being the preferred one.

In a preferred aspect, in the composition according to the present invention, the ibuprofen salt is ibuprofen L-arginine salt and the strong base is sodium carbonate. The pharmaceutically effective amount of a ibuprofen salt present in the compositions according to the present invention may vary, so long as it is effective to achieve the intended purpose of the composition, e.g., for the treatment of pain, in particular of pain related to the musculoskeletal system, but also headaches, postoperative pain, dysmenorrhea, etc.. In particular, the equivalent amount of ibuprofen contained in the compositions according to the invention may range from about 100 mg to about 800 mg.

Preferably, the compositions according to the present invention contain an amount of ibuprofen equal to 400 mg, 600 mg or 800 mg.

As already said above, a composition according to the invention comprises a mixture of a pharmaceutically effective amount of a ibuprofen salt and a pharmaceutically acceptable strong base in a molar ratio from 1:0.01 to 1:0.8, such that said pharmaceutical composition when dissolved a liquid solution, preferably an aqueous solution, more preferably drinkable water, affords a liquid solution having a pH in the range of from 9 to 9.5, preferably 9.2.

When the strong base selected from an alkaline metal carbonate is, sodium carbonate and the ibuprofen salt is sodium ibuprofen, the fact that the strong base is present in the pharmaceutical composition in molar defect to ibuprofen salt represents a great advantage to the subject in need thereof, because it helps to maintain said subject under the recommended daily sodium threshold, particularly when these subjects are under low sodium diet. Therefore, the compositions according to the present invention can be particularly appreciated not only by virtue of the removal of the burning sensation of the oral cavity, but also because they allow the preparation of drinkable compositions containing ibuprofen sodium salts containing the equivalent to high dosages of ibuprofen, having acceptable sodium content.

The composition of the invention can also optionally include one or more pharmaceutically acceptable excipients selected from, for example, preservative agents, diluting agents, sweetening agents, aromatic agents and artificial colors. According to the present invention, preferred excipients are selected from sodium saccharin, aspartame, sodium cyclamate, potassium acesulfame, fruits or cream flavor, sucrose, glucose, dextrose, lactose, sorbitol and maltodextrin.

In a preferred aspect of the present invention, a pharmaceutical non-effervescent solid composition comprises a mixture of a pharmaceutically effective amount of ibuprofen L-arginine salt, and sodium carbonate or sodium hydroxide in a molar ratio of from 1:0.5 to 1:0.8; said composition being such that, when dissolved in a suitable liquid for dilution, such as an aqueous solution, particularly drinkable water, imparts a pH value ranging from 9 to 9.5, preferably a pH value of 9.2 to a said liquid solution.

The compositions according to the invention are in the form of powder suitably contained in sachets. A sachet of the present invention usually contains an ibuprofen salt in an amount equivalent to a high oral unit dose of ibuprofen such as 800 mg 400 mg or 600 mg, preferably 400 mg or 600 mg (calculated as free ibuprofen).

The term sachet is used herein to describe any suitable container, package or bag to contain the pharmaceutical composition or formulation according to the present invention. Preferably the sachet is sealed, using any appropriate method. Preferably, the sachet is disposable. The sachet may be formed of any suitable material, including plastic, metal foil, paper or a combination thereof. The sachet may be provided with any suitable means for opening thereof, including a perforated region or a nick in the edge of the sachet for ease of tearing. The sachet may be of any suitable size. Preferably, the sachet is of a size to conveniently fit in a pocket, wallet or handbag. The sachet may be sold individually or as a collection of two or more sachets.

The present invention also provides kits, where said kits at least include one or more, e.g., a plurality of sachets, as described above. The sachets in the kits may be present in a package. The kits also generally include instructions for how to use the compositions.

A process for preparing a composition as described above can be carried out according to conventional techniques well known to a person skilled in the art. For example, the present invention also refers to a method of making a pharmaceutical, non-effervescent, solid oral composition comprising a pharmaceutically effective amount of an ibuprofen salt and a pharmaceutically acceptable strong base to impart a pH value ranging from 9 to 9.5 to a liquid solution of said composition, said method comprising:
(i) mixing together a pharmaceutically acceptable amount of an ibuprofen salt and a strong base so that the molar ratio of ibuprofen salt to a strong base may range from 1:0.01 to 1:0.8, preferably from 1:0.5 to 1:0.8;
(ii) preparing a solid oral composition by adding, optionally, pharmaceutically acceptable excipients;
(iii) filling said solid oral composition into a sachet; and, if desired,
(iv) inserting one or more sachets into a package.

It is a further object of the present invention a method of avoiding sensory irritation to the oral cavity caused by a ibuprofen salt selected from the group consisting of L-arginine, L-lysine, sodium and potassium ibuprofen salts when swallowed in a non-effervescent liquid pharmaceutical oral solution thereof, said method comprising mixing together said ibuprofen salt with a strong base selected from the group consisting of an alkaline metal carbonate such as, for example, sodium or potassium carbonate; an alkaline metal hydroxide such as, for example, sodium or potassium hydroxide; a tribasic metal phosphate such as, for example, sodium and potassium tribasic phosphate in a molar ratio of from 1:0.01 to 1:0.8, preferably from 1.0.5 to 1:0.8, with respect to ibuprofen salt, before dissolving said ibuprofen salt into a suitable liquid for dilution, preferably an aqueous solution, more preferably drinkable water.

In order to better illustrate the present invention without limiting it, the following examples are given below.

These examples are significant to demonstrate that, in order to achieve the removal of the throat irritation typically characterized as a sting, itch, or tickle associated with an ibuprofen salt, especially when the salt contains the equivalent to ibuprofen at high dosages in a liquid preparation, it is of primary importance to reach a pH ranging from 9.0 to 9.5 in the solution, irrespective of the molecules present in the solution, for example carbonate, sodium, potassium, arginine, lysine or other.

### Example 1

A pharmaceutical, non-effervescent, solid composition was prepared by mixing together all the components listed in the following table and filling them into unit dose sachets.

| **Component** | **mg** | **% w/w** |
|---|---|---|
| Ibuprofen L-arginine salt | 1105 (equivalent to 600 mg of ibuprofen) | 38,9 |
| Sodium carbonate | 230 | 7,7 |
| Sucrose | 1309 | 44,1 |
| Aspartame | 47 | 1,6 |
| Sodium saccharine | 47 | 1,6 |
| Lemon flavor | 180 | 6,1 |
| total | 2968 | 100,0 |

The aqueous solution obtained by dissolving 2,968 g of the resultant mixture in 50 ml of drinkable water at room temperature by manual stirring has pH = 9,21 measured with pHmeter Mettler Toledo model MA235.

The obtained solution was tested by a panel of 5 people who drank the solution to assess the effect on oral cavity irritation.

The assessment procedure required in order:
1) keep in mouth 10 ml of drinking water for 10 seconds followed by water swallowing,
2) keep in mouth 10 ml of drug solution for 10 seconds followed by swallowing. The panelist was required to report the instant throat irritation sensation after administration and after 5 minutes.

No panelist experienced irritation at throat or mouth level demonstrating the effectiveness of the composition according to the present invention.

### Example 2

A pharmaceutical, non-effervescent, solid composition was prepared by mixing together all the components listed in the following table and filling them into unit dose sachets.

| **Component** | **mg** | **% w/w** |
|---|---|---|
| Ibuprofen L-arginine salt | 1105 (equivalent to 600 mg of ibuprofen) | 43.0 |
| Sodium hydroxide | 68 | 2.5 |
| Sucrose | 1285 | 47.8 |
| Forest fruit | 180 | 6.7 |
| total | 2688 | 100,0 |

The aqueous solution obtained by dissolving 2,688 g of the resultant mixture in 50 ml of drinkable water at room temperature by manual stirring has pH= 9,23 measured with pHmeter Mettler Toledo model MA235.

The obtained solution was tested by a panel of 5 people who drank the solution to assess the effect on oral cavity irritation following the same procedure reported in Example 1.

No panelist experienced irritation at throat or mouth level.

### Example 3

A pharmaceutical, non-effervescent, solid composition was prepared by mixing together all the components listed in the following table and filling them into unit dose sachets.

| **Component** | **mg** | **% w/w** |
|---|---|---|
| Ibuprofen sodium salt | 664 (equivalent to 600 mg of ibuprofen) | 31.0 |
| Sodium carbonate | 10 | 0.5 |
| Sucrose | 1285 | 60.1 |
| Orange flavor | 180 | 8.4 |
| total | 2139 | 100,0 |

The aqueous solution obtained by dissolving 2,139 g of the resultant mixture in 50 ml of drinkable water at room temperature by manual stirring has pH= 9,21 measured with pHmeter Mettler Toledo model MA235.

The obtained solution was tested by a panel of 4 people who drank the solution to assess the effect on oral cavity irritation following the same procedure reported in Example 1.

No panelist experienced irritation at throat or mouth level.

### Reference Example 4

A pharmaceutical, non-effervescent, solid composition was prepared by mixing together all the components listed in the following table and filling them into unit dose sachets.

| **Component** | **mg** | **% w/w** |
|---|---|---|
| Ibuprofen Sodium salt | 664 (equivalent to 600 mg of ibuprofen) | 31.2 |
| Sucrose | 1285 | 60.3 |
| Apricot flavor | 180 | 8.5 |
| total | 2129 | 100,0 |

The aqueous solution obtained by dissolving 2.129 g of the resultant mixture in 50 ml of drinkable water at room temperature by manual stirring has pH= 7.93 measured with pHmeter Mettler Toledo model MA235.

The obtained solution was tested by a panel of 5 people who drank the solution to assess the effect on oral cavity irritation following the same procedure reported in Example 1.

All panelist experience intense irritation at throat level similar to example 4, this sensation appears at the moment of taking it and remains for an extended period.

### Reference Example 5

A pharmaceutical, non-effervescent, solid composition was prepared by mixing together all the components listed in the following table and filling them into unit dose sachets. The weak base sodium hydrogen carbonate was added instead of the strong base sodium carbonate.

| **Component** | **mg** | **% w/w** |
|---|---|---|
| Ibuprofen L-arginine salt | 1105 (equivalent to 600 mg of ibuprofen) | 41.7 |
| Sodium hydrogen carbonate | 183 (the same molars of sodium carbonate of Example 1) | 6.6 |
| Sucrose | 1253 | 45.2 |
| Forest fruit | 180 | 6.5 |
| total | 2771 | 100,0 |

The aqueous solution obtained by dissolving 2,771 g of the resultant mixture in 50 ml of drinkable water at room temperature by manual stirring has pH= 8,01 measured with pHmeter Mettler Toledo model MA235.

The obtained solution was tested by a panel of 3 people who drank the solution to assess the effect on oral cavity irritation following the same procedure reported in Example 1.

All panelist experience intense irritation at throat level, which appears at the moment of taking it and remains for an extended period.

## Claims

1. A pharmaceutical, non-effervescent, solid composition comprising a mixture of a pharmaceutically effective amount of a ibuprofen salt and a pharmaceutically acceptable strong base in a molar ratio of from 1:0.01 to 1:0.8, wherein said pharmaceutically effective amount of ibuprofen salt is from 100 mg to 800 mg expressed as equivalent amount of ibuprofen, said composition being such that, when dissolved in drinkable water having a volume of from 25 ml to 100 ml, imparts a pH value ranging from 9.0 to 9.5 to the obtained solution.

2. The composition according to claim 1, wherein a mixture of a pharmaceutically effective amount of the ibuprofen salt and the pharmaceutically acceptable strong base is in a molar ratio of from 1:0.5 to 1:0.8.

3. The composition according to anyone of the preceding claims, wherein the pH value is 9.2.

4. The composition according to anyone of the preceding claims, wherein the ibuprofen salt is selected from L-arginine, L-lysine, sodium and potassium ibuprofen salt.

5. The composition according to claim 4, wherein the ibuprofen salt is ibuprofen L-arginine.

6. The composition according to anyone of the preceding claims, wherein the strong base is selected from an alkaline metal carbonate, an alkaline metal hydroxide and a tribasic metal phosphate.

7. The composition according to claim 6, wherein the alkaline metal carbonate is sodium carbonate.

8. The composition according to claim 6, wherein the alkaline metal hydroxide is sodium hydroxide.

9. The compositions according to anyone of the preceding claims, wherein the composition is in the form of powder suitably contained in sachets.

10. The composition according to claim 9, wherein the sachet contains an ibuprofen salt in an amount equivalent to a conventional oral unit dose of ibuprofen.

11. The composition according to claim 10 wherein the conventional oral unit dose of ibuprofen is equal to 400 mg, 600 mg or 800 mg.

12. The use of a composition according to claim 1, for preparing an aqueous solution having a pH value ranging from 9 to 9.5, wherein said composition is dissolvable in drinkable water having a volume of from 25 ml to 100 ml.

13. The use according to claim 12, wherein the drinkable water has a volume of 50 ml.

14. A non-irritant liquid formulation obtainable by dissolving the composition according to claim 1 in drinkable water having a volume of from 25 ml to 100 ml.

15. A non-irritant liquid formulation for use in avoiding sensory irritation to the oral cavity caused by a ibuprofen salt selected from the group consisting of L-arginine, L-lysine, sodium and potassium ibuprofen salts when swallowed in a non-effervescent liquid pharmaceutical oral solution thereof, said non-irritant liquid formulation being obtained by: mixing together a pharmaceutically effective amount of said ibuprofen salt with a pharmaceutically acceptable strong base selected from the group consisting of sodium carbonate, potassium carbonate; sodium hydroxide, potassium hydroxide; sodium tribasic phosphate and potassium tribasic phosphate in a molar ratio of from 1:0.01 to 1:0.8, with respect to ibuprofen salt before dissolving and then dissolving the resulting mixture in drinkable water.

16. The non-irritant liquid formulation according to claim 15 wherein the drinkable water has a volume of from 25 ml to 100 ml.

17. A non-irritant liquid formulation for use in eliminating unpleasant feeling of irritation of the oral cavity that usually follows the ingestion of a ibuprofen salt as a liquid solution, said liquid formulation being obtained by dissolving a composition according to claim 1 in drinkable water having a volume of from 25 ml to 100 ml.

18. A method of making a pharmaceutical, non-effervescent, solid oral composition comprising a pharmaceutically effective amount of a ibuprofen salt and a pharmaceutically acceptable strong base to impart a pH value ranging from 9 to 9.5 to a drinkable water solution of said composition having a volume of from 25 ml to 100 ml, said method comprising:
(i) mixing together a pharmaceutically acceptable amount of an ibuprofen salt and a strong base so that the molar ratio of the ibuprofen salt to a strong base may range from 1:0.01 to 1:0.8, wherein said pharmaceutically acceptable amount of ibuprofen salt is from 100 mg to 800 mg expressed as equivalent amount of ibuprofen;
(ii) preparing a solid oral composition by adding pharmaceutically acceptable excipients; and
(iii) filling said solid oral composition into a sachet.

19. The method according to claim 18, wherein said method comprises a further step (iv) after step (iii), and wherein said further step (iv) comprises inserting one or more of said sachets into a package.

20. The pharmaceutical non-effervescent solid composition according to claim 1, comprising a mixture of a pharmaceutically effective amount of ibuprofen L-arginine salt, and sodium carbonate or sodium hydroxide in a molar ratio of from 1:0.5 to 1:0.8.

## Patentansprüche

1. Feste pharmazeutische nichtsprudelnde Zusammensetzung, umfassend ein Gemisch einer pharmazeutisch wirksamen Menge eines Ibuprofen-Salzes und einer pharmazeutisch annehmbaren starken Base in einem Molverhältnis von 1 : 0,01 bis 1: 0,8, wobei die in Ibuprofen-Äquivalenten ausgedrückte pharmazeutisch wirksame Menge des Ibuprofen-Salzes von 100 mg bis 800 mg beträgt und die Zusammensetzung so beschaffen ist, dass sie beim Auflösen in Trinkwasser eines Volumens von 25 ml bis 100 ml der erhaltenen Lösung einen pH-Wert im Bereich von 9,0 bis 9,5 verleiht.

2. Zusammensetzung nach Anspruch 1, wobei ein Gemisch einer pharmazeutisch wirksamen Menge des Ibuprofen-Salzes und der pharmazeutisch annehmbaren starken Base ein Molverhältnis von 1 : 0,5 bis 1 : 0,8 aufweist.

3. Zusammensetzung nach einem der vorhergehenden Ansprüche, wobei der pH-Wert 9,2 beträgt.

4. Zusammensetzung nach einem der vorhergehenden Ansprüche, wobei das Ibuprofen-Salz unter dem L-Arginin-, L-Lysin-, Natrium- und Kalium-Salz des Ibuprofens ausgewählt ist.

5. Zusammensetzung nach Anspruch 4, wobei das Ibuprofen-Salz das L-Arginin-Salz des Ibuprofens ist.

6. Zusammensetzung nach einem der vorhergehenden Ansprüche, wobei die starke Base unter einem Alkalimetallcarbonat, einem Alkalimetallhydroxid und einem dreibasischen Metallphosphat ausgewählt ist.

7. Zusammensetzung nach Anspruch 6, wobei das Alkalimetallcarbonat Natriumcarbonat ist.

8. Zusammensetzung nach Anspruch 6, wobei das Alkalimetallhydroxid Natriumhydroxid ist.

9. Zusammensetzung nach einem der vorhergehenden Ansprüche, wobei die Zusammensetzung in der Form eines Pulvers vorliegt, das geeigneterweise in Sachets enthalten ist.

10. Zusammensetzung nach Anspruch 9, wobei das Sachet ein Ibuprofen-Salz in einer Menge enthält, die einer üblichen oralen Einzeldosis Ibuprofen entspricht.

11. Zusammensetzung nach Anspruch 10, wobei die übliche orale Einzeldosis Ibuprofen 400 mg, 600 mg oder 800 mg entspricht.

12. Verwendung einer Zusammensetzung nach Anspruch 1 zur Herstellung einer wässrigen Lösung mit einem pH-Wert im Bereich von 9 bis 9,5, wobei die Zusammensetzung in Trinkwasser mit einem Volumen von 25 ml bis 100 ml löslich ist.

13. Verwendung nach Anspruch 12, wobei das Trinkwasser ein Volumen von 50 ml aufweist.

14. Nicht-reizende flüssige Formulierung, erhältlich durch Lösen der Zusammensetzung nach Anspruch 1 in Trinkwasser mit einem Volumen von 25 ml bis 100 ml.

15. Nicht-reizende flüssige Formulierung zur Verwendung, um die durch ein Ibuprofen-Salz, das unter L-Arginin-, L-Lysin-, Natrium- und Kalium-Salzen des Ibuprofens ausgewählt ist, beim Schlucken seiner flüssigen pharmazeutischen Lösung ausgelöste sensorische Reizwirkung auf die Mundhöhle zu vermeiden, wobei man die nicht-reizende flüssige Formulierung erhält, indem man eine pharmazeutisch wirksame Menge des Ibuprofen-Salzes mit einer unter Natriumcarbonat, Kaliumcarbonat; Natriumhydroxid, Kaliumhydroxid, dreibasischem Natriumphosphat und dreibasischem Kaliumphosphat ausgewählten pharmazeutisch annehmbaren starken Base in einem Molverhältnis von 1 : 0,01 bis 1 : 0,8 bezüglich des Ibuprofen-Salzes vor dem Lösen mischt, und das erhaltene Gemisch dann in Trinkwasser löst.

16. Nicht-reizende flüssige Formulierung nach Anspruch 15, wobei das Trinkwasser ein Volumen von 25 ml bis 100 ml aufweist.

17. Nicht-reizende flüssige Formulierung zur Verwendung, um ein unangenehmes Gefühl der Irritation der Mundhöhle, das der Einnahme eines Ibuprofen-Salzes als flüssige Lösung normalerweise folgt, zu unterdrücken, wobei man die flüssige Formulierung erhält, indem man eine Zusammensetzung nach Anspruch 1 in Trinkwasser mit einem Volumen von 25 ml bis 100 ml löst.

18. Verfahren zur Herstellung einer festen pharmazeutischen nichtsprudelnden Zusammensetzung zur oralen Verabreichung, die eine pharmazeutisch wirksame Menge eines Ibuprofen-Salzes und eine pharmazeutisch annehmbare starke Base enthält, um einen pH-Wert im Bereich von 9 bis 9,5 in einer ein Volumen von 25 ml bis 100 ml aufweisenden Trinkwasserlösung der Zusammensetzung hervorzurufen, wobei man:
(i) eine pharmazeutisch annehmbare Menge eines Ibuprofen-Salzes und eine starke Base mischt, so dass das Molverhältnis des Ibuprofen-Salzes zur starken Base im Bereich von 1 : 0,01 bis 1 : 0,8 liegt, wobei die in Ibuprofen-Äquivalenten ausgedrückte pharmazeutisch annehmbare Menge des Ibuprofen-Salzes von 100 mg bis 800 mg beträgt;
(ii) eine feste Zusammensetzung zur oralen Verabreichung durch Zugabe pharmazeutisch annehmbarer Hilfsstoffe herstellt; und
(iii) die feste Zusammensetzung zur oralen Verabreichung in ein Sachet füllt.

19. Verfahren nach Anspruch 18, wobei das Verfahren einen weiteren Schritt (iv) nach Schritt (iii) umfasst, und man bei dem weiteren Schritt (iv) eine oder mehrere Sachets in eine Verpackung einbringt.

20. Feste pharmazeutische nichtsprudelnde Zusammensetzung nach Anspruch 1, umfassend ein Gemisch einer pharmazeutisch wirksamen Menge des L-Arginin-Salzes des Ibuprofens und Natriumcarbonat oder Natriumhydroxid in einem Molverhältnis von 1 : 0,5 bis 1:0,8.

## Revendications

1. Composition pharmaceutique solide, non effervescente comprenant un mélange d'une quantité pharmaceutiquement efficace d'un sel d'ibuprofène et d'une base forte pharmaceutiquement acceptable dans un rapport molaire de 1:0,01 à 1:0,8, dans laquelle ladite quantité pharmaceutiquement efficace de sel d'ibuprofène est de 100 mg à 800 mg exprimée en quantité équivalente d'ibuprofène, ladite composition étant telle que, lorsqu'elle est dissoute dans de l'eau potable ayant un volume de 25 ml à 100 ml, confère une valeur de pH dans la plage de 9,0 à 9,5 à la solution obtenue.

2. Composition selon la revendication 1, dans laquelle un mélange d'une quantité pharmaceutiquement efficace du sel d'ibuprofène et la base forte pharmaceutiquement acceptable est dans un rapport molaire de 1:0,5 à 1:0,8.

3. Composition selon l'une quelconque des revendications précédentes, dans laquelle la valeur de pH est de 9,2.

4. Composition selon l'une quelconque des revendications précédentes, dans laquelle le sel d'ibuprofène est choisi parmi le sel d'ibuprofène de L-arginine, de L-lysine, de sodium et de potassium.

5. Composition selon la revendication 4, dans laquelle le sel d'ibuprofène est le sel de L-arginine d'ibuprofène.

6. Composition selon l'une quelconque des revendications précédentes, dans laquelle la base forte est choisie parmi un carbonate de métal alcalin, un hydroxyde de métal alcalin et un phosphate de métal tribasique.

7. Composition selon la revendication 6, dans laquelle le carbonate de métal alcalin est le carbonate de sodium.

8. Composition selon la revendication 6, dans laquelle l'hydroxyde de métal alcalin est l'hydroxyde de sodium.

9. Compositions selon l'une quelconque des revendications précédentes, dans lesquelles la composition est sous la forme de poudre contenue de manière appropriée dans des sachets.

10. Composition selon la revendication 9, dans laquelle le sachet contient un sel d'ibuprofène en une quantité équivalente à une dose unitaire orale d'ibuprofène conventionnelle.

11. Composition selon la revendication 10 dans laquelle la dose unitaire orale d'ibuprofène conventionnelle est égale à 400 mg, 600 mg ou 800 mg.

12. Utilisation d'une composition selon la revendication 1, pour préparer une solution aqueuse ayant une valeur de pH dans la plage de 9 à 9,5, dans laquelle ladite composition peut être dissoute dans de l'eau potable ayant un volume de 25 ml à 100 ml.

13. Utilisation selon la revendication 12, dans laquelle l'eau potable a un volume de 50 ml.

14. Formulation liquide non irritante pouvant être obtenue par dissolution de la composition selon la revendication 1 dans de l'eau potable ayant un volume de 25 ml à 100 ml.

15. Formulation liquide non irritante pour utilisation dans la prévention d'une irritation sensorielle de la cavité buccale causée par un sel d'ibuprofène choisi dans le groupe constitué des sels d'ibuprofène de L-arginine, de L-lysine, de sodium et de potassium lorsqu'elle est avalée dans une solution pharmaceutique orale liquide non effervescente de celle-ci, ladite formulation liquide non irritante étant obtenue par : mélange conjointement d'une quantité pharmaceutiquement efficace dudit sel d'ibuprofène avec une base forte pharmaceutiquement acceptable choisie dans le groupe constitué du carbonate de sodium, du carbonate de potassium ; de l'hydroxyde de sodium, de l'hydroxyde de potassium ; du phosphate tribasique de sodium et du phosphate tribasique de potassium dans un rapport molaire de 1:0,01 à 1:0,8, par rapport au sel d'ibuprofène avant dissolution et ensuite dissolution du mélange résultant dans de l'eau potable.

16. Formulation liquide non irritante selon la revendication 15 dans laquelle l'eau potable a un volume de 25 ml à 100 ml.

17. Formulation liquide non irritante pour utilisation dans l'élimination de la sensation déplaisante d'irritation de la cavité buccale qui suit généralement l'ingestion d'un sel d'ibuprofène sous la forme d'une solution liquide, ladite formulation liquide étant obtenue par dissolution d'une composition selon la revendication 1 dans de l'eau potable ayant un volume de 25 ml à 100 ml.

18. Procédé de fabrication d'une composition pharmaceutique, orale, solide, non effervescente comprenant une quantité pharmaceutiquement efficace d'un sel d'ibuprofène et une base forte pharmaceutiquement acceptable pour conférer une valeur de pH dans la plage de 9 à 9,5 à une solution dans de l'eau potable de ladite composition ayant un volume de 25 ml à 100 ml, ledit procédé comprenant :
(i) le mélange conjointement d'une quantité pharmaceutiquement acceptable d'un sel d'ibuprofène et d'une base forte de sorte que le rapport molaire du sel d'ibuprofène à une base forte puisse être dans la plage de 1:0,01 à 1:0,8, ladite quantité pharmaceutiquement acceptable de sel d'ibuprofène étant de 100 mg à 800 mg exprimée en quantité équivalente d'ibuprofène ;
(ii) la préparation d'une composition orale solide par ajout d'excipients pharmaceutiquement acceptables ; et
(iii) le remplissage de ladite composition orale solide dans un sachet.

19. Procédé selon la revendication 18, ledit procédé comprenant une étape supplémentaire (iv) après l'étape (iii), et dans lequel ladite étape supplémentaire (iv) comprend l'insertion d'un ou plusieurs desdits sachets dans un emballage.

20. Composition pharmaceutique, solide, non effervescente selon la revendication 1, comprenant un mélange d'une quantité pharmaceutiquement efficace de sel de L-arginine d'ibuprofène, et de carbonate sodium ou d'hydroxyde de sodium dans un rapport molaire de 1:0,5 à 1:0,8.
